# EUROPÄISCHE PATENTSCHRIFT

(11) **EP 3 624 749 B1**
(45) Veröffentlichungstag und Bekanntmachung des Hinweises auf die Patenterteilung: **07.04.2021**
(21) Anmeldenummer: 18726035.1
(22) Anmeldetag: 02.05.2018
(51) Int. Cl.: A61G 13/00, A61G 13/12, A61F 2/46, A61B 90/00

(54) **BEINZUGVORRICHTUNG**
LEG PULLING DEVICE
DISPOSITIF DE TRACTION DE JAMBE

(30) Priorität: 17.05.2017 DE 102017208306
(43) Veröffentlichungstag der Anmeldung: 25.03.2020
(73) Patentinhaber: Condor Medtec GmbH, 33154 Salzkotten (DE)
(72) Erfinder: WEIGEL, Lothar, 33034 Brakel (DE); SCHULTE, Dominik, 33154 Salzkotten (DE); HENKE, Christian, 32839 Steinheim (DE)
(74) Vertreter: Rolf, Gudrun
(86) Internationale Anmeldenummer: PCT/DE2018/100417
(87) Internationale Veröffentlichungsnummer: WO 2018/210375

(56) Entgegenhaltungen:
- EP-A1- 1 364 636
- DE-A1-102011 051 937
- DE-U1-202012 101 347
- DE-U1-202012 104 964

## Beschreibung

Die Erfindung betrifft eine Beinzugvorrichtung mit einer Zugvorrichtung und einer Stützvorrichtung, wobei mindestens eine Kraft oder zumindest ein Druck messbar ist, die oder der von der Stützvorrichtung bei Betätigung der Zugvorrichtung auf einen Patienten ausgeübt wird.

Bei vielfältigen Einsätzen im Rahmen von hüftendoprothetischen, hüftarthroskopischen und traumatologischen Eingriffen ist sowohl eine exakte Beinpositionierung wie auch Beinfixierung von enormer Wichtigkeit. Eine eigenständige Justierung dieser beiden Parameter ermöglicht dem ausführenden Operateur ein schnelleres, präziseres und dadurch nicht zuletzt ein kosteneffizienteres Arbeiten am Hüftgelenk eines Patienten. Zusätzlich kann zur optimalen Lagerung des Patienten, beispielsweise bei intraoperativen Röntgenkontrollen ohne Sichtbehinderung, eine Extension der unteren Extremitäten hilfreich bzw. notwendig sein. Die beschriebenen Funktionalitäten bieten nur wenige am Markt befindliche OP-Tischsysteme. Diese ermöglichen zwar dem Operateur eine Beinpositionierung, Fixierung und ggfs. Extension in den erforderlichen Freiheitsgraden, geben ihm aber keine Auskunft über die dabei auftretenden Kräfte und eventuell anatomisch nicht hinnehmbare Lagen der unteren Extremitäten. Dadurch kann es zu Quetschungen, Hämatomen und sonstigen Schäden am Patienten kommen, die als unerwünschte Nebenwirkungen oder gar Spätfolgen eines operativen Eingriffs seine Lebensqualität nicht unerheblich beeinträchtigen.

Aus dem Stand der Technik ist eine Beinzugvorrichtung bekannt, bei welcher ein Patient an einem Operationstisch positioniert wird, so dass sich eine Stützrolle zwischen seinen Oberschenkelknochen befindet und der Patient mit seinem unteren Beckenbereich gegen die Stützrolle abgestützt werden kann. Ein Fuß des Patienten wird in einem Extensionsschuh fixiert. Der Extensionsschuh ist an einer Gewindespindel drehbar um eine Spindelachse gelagert, so dass auch die im Extensionsschuh fixierte Extremität um dieselbe Achse gedreht und in der erforderlichen Winkelposition arretiert werden kann. Mit der Gewindespindel ist eine Zugkraft auf das Bein des Patienten ausübbar. Die Zugkraft kann durch Drehen einer Handkurbel an der Spindel erzeugt werden. Die Zugkraft wird in Richtung der Spindelachse erzeugt, wodurch ein Schenkelkopf des Patienten aus dessen Hüftpfanne gehoben werden kann und die Sicht auf die betroffenen Stellen, wie beispielweise Gelenkknorpel, freigegeben werden kann.

Problematisch bei der Vorrichtung des Standes der Technik ist, dass bei Überschreitung der kritischen Zugkräfte dem Patienten Quetschungen der an der Stützrolle abstützenden Weichteile, Hämatome und sonstige Schäden zugeführt werden können.

Bekannt ist des Weiteren eine Vorrichtung zur Durchführung von Operationen an extendierten Extremitäten, DE 20 2012 104 964 U1, die zwar eine Druckmessmatte an einer Stützvorrichtung im Schritt eines Patienten aufweist, ebenso wie eine Positioniervorrichtung eines in einer Fußaufnahme einer Fußrotationsvorrichtung gelagerten Patientenbeines eine Stützvorrichtung aufweist, DE 20 2012 101 347, deren Stützvorrichtungen allerdings beide ortsfest angeordnet sind, sodass es auch hier zu unerwünschten Verletzungen oder anderen Schäden kommen kann.

Aufgabe der vorliegenden Erfindung ist es, eine Beinzugvorrichtung anzugeben, mit der derartige Schäden vermieden werden können.

Die Aufgabe wird gelöst durch die Beinzugvorrichtung nach Anspruch 1. Die abhängigen Ansprüche geben vorteilhafte Weiterbildungen der erfindungsgemäßen Beinzugvorrichtung an.

Erfindungsgemäß wird eine Beinzugvorrichtung angegeben, die zum Einen eine Zugvorrichtung aufweist, mit der eine Zugkraft auf ein Bein eines Patienten ausübbar ist und zum Anderen eine Stützvorrichtung, die so gegenüber der Zugvorrichtung angeordnet ist, dass die Stützvorrichtung den Patienten gegen die von der Zugvorrichtung ausgeübte Zugkraft abstützen kann. Unabhängig davon, wie die Zugvorrichtung und die Stützvorrichtung konkret ausgestaltet sind, tritt in dieser Konstellation das durch die Erfindung angegangene Problem auf, dass der Patient durch den Abstützvorgang an der Stützvorrichtung Schaden erleiden kann. Die Beinzugvorrichtung kann optional auch als Beinzugmessvorrichtung bezeichnet werden oder eine solche sein.

Die Zugvorrichtung kann so gegenüber der Stützvorrichtung angeordnet sein, dass eine durch die Zugvorrichtung erzeugbare Kraft eine Komponente hat, die direkt auf die Stützvorrichtung hingerichtet ist. Im Allgemeinen wird jedoch die durch die Zugvorrichtung ausgeübte Kraft selbst nicht direkt auf die Stützvorrichtung gerichtet sein, sondern an dieser vorbeiweisen, da im Einsatz die von der Zugvorrichtung ausgeübte Kraft über den Körper des Patienten auf die Stützvorrichtung geleitet wird.

Erfindungsgemäß ist die Beinzugvorrichtung auf einem Operationstisch anordbar. Eine Liegefläche des Operationstisches kann, muss aber nicht, als Teil der Beinzugvorrichtung angesehen werden. Die Stützvorrichtung ist vorteilhaft auf der Liegefläche des Operationstisches angeordnet, auf der im Anwendungsfall der Patient liegt.

Besonders vorteilhaft ist dabei, dass die Stützvorrichtung mittels zweier zueinander senkrechter Führungsschlitten auf der Liegefläche lagerbar ist. Hierzu kann beispielsweise die Stützvorrichtung selbst auf einem ersten Schlitten gelagert sein, der eine Verschiebung der Stützvorrichtung in einer ersten Richtung ermöglicht, und die Stützvorrichtung zusammen mit dem ersten Schlitten kann auf einem zweiten Schlitten gelagert sein, der eine Bewegung der Stützvorrichtung und des ersten Schlittens in einer zur ersten Richtung senkrechten zweiten Richtung erlaubt. Vorteilhafterweise können die erste Richtung und die zweite Richtung mit den zueinander senkrechten Richtungen übereinstimmen, in welchen mit den vorstehend beschriebenen zwei Kraftsensoren Kräfte messbar sind.

Die Zugvorrichtung kann vorteilhafterweise eine Kraft ausüben, die eine Komponente in Richtung parallel zur Liegefläche des Operationstisches hat, auf der der Patient im Einsatzfall liegt. Diese Komponente der Zugkraft kann vorteilhaft mit der Zugkraft selbst identisch sein oder die größte Komponente der Zugkraft darstellen. Die in Richtung parallel zur Oberfläche des Operationstisches liegende Kraftkomponente ist jene, die für die Ausführung der Operation entscheidend ist.

Erfindungsgemäß weist die Stützvorrichtung und/oder die Zugvorrichtung zumindest einen Sensor auf, mit dem zumindest eine Kraft oder zumindest ein Druck messbar ist, welche Kraft oder welcher Druck von der Stützvorrichtung durch Betätigung der Zugvorrichtung auf den Patienten ausgeübt wird. Es sei darauf hingewiesen, dass die erfindungsgemäße Aufgabe durch beide Alternativen lösbar ist, sowohl Kraftsensoren als auch Drucksensoren. Vorteilhafterweise sollen Drucksensoren hier als besondere Ausführungsformen von Kraftsensoren angesehen werden, da sie ebenfalls Kräfte, jedoch pro Fläche, messen.

Sensoren, die lediglich eine Kraft, jedoch keinen Druck messen, sind einfach und kostengünstig realisierbar. Andererseits ermöglichen Drucksensoren genauere Messungen der tatsächlich auf den Patienten wirkenden Drücke. In vielen Fällen ist ein Kraftsensor, mit dem nicht direkt ein Druck gemessen wird, jedoch ausreichend, da von gemessenen Kräften auf die Drücke schließbar ist, die auf den Patienten wirken. Dabei kann beispielsweise von einer Bandbreite an anatomischen Formen des Patienten ausgegangen werden und die Zugkraft so begrenzt werden, dass sie bei keiner der anatomischen Gegebenheiten zu Schädigungen des Patienten führt. Vorteilhaft kann eine Datenbank vorgesehen sein, die kritische Werte für unterschiedliche Personengruppen wie z.B. Kinder, Erwachsene, Männer, Frauen etc. enthält. Auch für unterschiedliche Körperkonstitutionen können Werte gespeichert werden.

In einer vorteilhaften Ausgestaltung der Erfindung kann die Stützvorrichtung den zumindest einen Druck- oder Kraftsensor aufweisen. In einer besonders vorteilhaften Ausgestaltung kann der Sensor eine Druckmessfolie aufweisen oder sein, die auf jenen Oberflächen der Stützvorrichtung angeordnet ist, die auf den Körper des Patienten drücken, wenn mittels der Zugvorrichtung eine Zugkraft auf das Bein des Patienten ausgeübt wird. Durch Einsatz der Druckmessfolie an der Stützvorrichtung können die genauesten Ergebnisse erzielt werden.

In einer vorteilhaften Ausgestaltung der Erfindung kann die Stützvorrichtung zumindest zwei der Sensoren aufweisen, mit denen zwei zueinander senkrechte Kraftkomponenten in Richtung parallel zur Liegefläche messbar sind. In dieser Ausgestaltung misst also ein Sensor eine auf die Stützvorrichtung wirkende Kraftkomponente in einer Richtung und ein anderer Sensor eine auf die Stützvorrichtung wirkende Kraftkomponente in hierzu senkrechter Richtung.

In einer vorteilhaften Ausgestaltung der Erfindung kann die Stützvorrichtung eine Stützrolle aufweisen oder sein, die mit ihrer Rollenachse senkrecht auf einer Liegefläche steht, auf der der Patient während der Operation liegt, z.B. der Oberfläche des Operationstisches. Dabei kann die Rolle so angeordnet sein, dass sie bei bestimmungsgemäßer Verwendung zwischen den Oberschenkelknochen des Patienten liegt, wenn mit der Zugvorrichtung die Zugkraft auf das Bein des Patienten ausgeübt wird. Auf diese Weise stützt die Rolle den Patienten gegen diese Zugkraft ab. Die beschriebene Druckmessfolie kann ggfs. auf der Oberfläche dieser Rolle angeordnet sein.

Die Zugvorrichtung weist vorteilhafterweise eine Fixierungsvorrichtung zur Fixierung eines Fußes des Patienten auf, wenn die Zugkraft auf das Bein als Extremität ausgeübt wird. Die Fixierungsvorrichtung kann beispielsweise als Extensionsschuh ausgestaltet sein. Die Zugvorrichtung kann dann die Fixierungsvorrichtung in Richtung einer Längsrichtung des Beines des Patienten ziehen.

In einer vorteilhaften Ausgestaltung kann die Fixierungsvorrichtung einen Neigungssensor aufweisen, mit dem die Lage der Fixierungsvorrichtung um zumindest eine Achse, vorzugsweise um drei Achsen messbar ist. Auf diese Weise kann die genaue Lage des betroffenen Beines im Raum erfasst werden.

Die Zugvorrichtung kann vorteilhafterweise eine Zugspindel aufweisen, mit der die Zugkraft auf die Extremität des Patienten einstellbar ist. Die Zugspindel kann beispielsweise eine Gewindespindel sein, die durch einen Operateur mittels einer Kurbel bedienbar ist. Die Zugkraft kann dabei in Richtung der Spindelachse ausgeübt werden.

Vorteilhafterweise kann die erfindungsgemäße Beinzugvorrichtung eine Mikrocontroller-Einheit aufweisen, mit welcher durch die Kraft- oder Drucksensoren und ggfs. auch durch den Neigungssensor erzeugte Messwerte auswertbar sind, und mit welcher bei Überschreitung von kritischen Schwellenwerten der mit dem Kraftsensoren gemessenen Kräfte, der mit dem Drucksensor gemessenen Drücke und/oder der mit dem Neigungssensor gemessenen Neigungen ein Alarmsignal erzeugbar ist. Derartige Schwellenwerte können beispielsweise ab Werk vorgegeben werden oder unter Berücksichtigung der gegebenen Operationsbedingungen vorab experimentell bestimmt werden. Insbesondere können sie in oben beschriebener Datenbank hinterlegt werden. Im Folgenden soll die Erfindung anhand einer Figuren beispielhaft erläutert werden. Gleiche Bezugszeichen kennzeichnen dabei gleiche oder entsprechende Merkmale. Die in den Beispielen beschriebenen Merkmale können auch unabhängig vom konkreten Beispiel realisiert sein und unter den verschiedenen Beispielen kombiniert werden.

Es zeigt:
- Figur 1:: eine beispielhafte Ausgestaltung einer erfindungsgemäßen Beinzugvorrichtung,
- Figur 2:: eine beispielhafte Lagerung einer Stützvorrichtung und
- Figur 3:: eine weitere beispielhafte Ausgestaltung einer erfindungsgemäßen Beinzugvorrichtung.

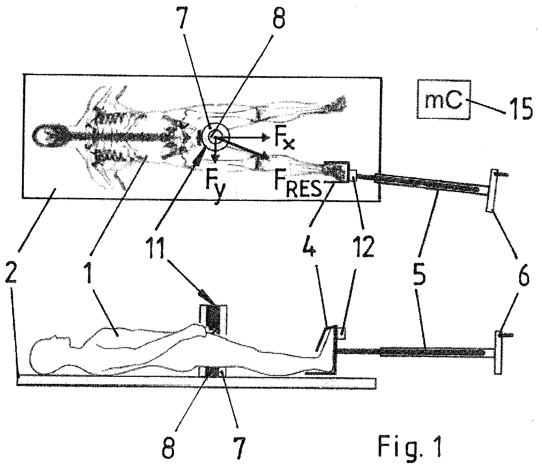

Figur 1 zeigt eine erste beispielhafte Ausgestaltung einer erfindungsgemäßen Beinzugvorrichtung in der Anwendung auf einen Patienten 1. Die Beinzugvorrichtung weist eine Zugvorrichtung 5 auf, hier eine Zugspindel, und eine Stützvorrichtung 11, hier eine Stützrolle. Die Stützrolle der Stützvorrichtung 11 ist so gegenüber der Zugvorrichtung 5 angeordnet, dass die Stützvorrichtung 11 den Patienten 1 gegen die von der Zugvorrichtung 5 ausgeübte Zugkraft abstützt.

Der Patient 1 liegt auf einer Liegefläche 2, hier der Oberfläche eines Operationstisches. Der Patient liegt auf dem Rücken und die Stützvorrichtung ist zwischen seinen Oberschenkeln angeordnet. Der rechte Fuß des Patienten ist in einem Extensionsschuh 4 fixiert, der mit der Zugvorrichtung 5 verbunden ist. Auf diese Weise ist mit der Zugvorrichtung 5 eine Zugkraft in einer Längsrichtung der Zugvorrichtung 5 auf den Extensionsschuh 4 und damit auf das Bein des Patienten 1 ausübbar.

Im gezeigten Beispiel weist die Stützvorrichtung 11 zwei Kraftsensoren 13;14 auf, mit denen Kraftkomponenten Fₓ und F_{y} messbar sind, so dass aus den Kraftkomponenten aus eine resultierende Kraft F**_{RES}** berechenbar ist. Die durch die Zugvorrichtung 5 ausgeübte Kraft ist, da die Zugvorrichtung 5 hier als Gewindespindel ausgestaltet ist, mittels einer Kurbel 6 einstellbar, die um die Längsachse der Zugvorrichtung 5 drehbar ist.

Am Extensionsschuh 4 ist im gezeigten Beispiel ein Neigungssensor 12 angeordnet, mit dem eine Neigung des Extensionsschuhs 4 und damit des Beines des Patienten 1 um eine, zwei oder drei Achsen bestimmbar ist. Die gezeigte Vorrichtung weist außerdem eine Mikrocontroller-Einheit 15 auf, mit der die Messungen der Kraftsensoren 13;14 und Neigungssensoren 12 auswertbar sind.

Mit der in Figur 1 gezeigten Ausgestaltung der erfindungsgemäßen Beinzugvorrichtung kann die Zugkraft und die Lage der betroffenen Extremität im Raum bestimmt werden. Der Kraftvektor F**_{RES}** kann mittels der Mikrocontroller-Einheit 15 bestimmt werden. Die Mikrocontroller-Einheit 15 erfasst die Werte und kann einem Operateur ein Warnsignal geben, wenn kritische Schwellenwerte erreicht werden. Entsprechend kann die Controller-Einheit 15 auch ein Warnsignal an den Operateur geben, wenn ein mittels des Neigungssensors 12 ermittelter Stellwinkel eine aus anatomischer Sicht unzulässige Lage der Extremität zur Folge hat.

Vorteilhaft kann in der Mikrocontroller-Einheit 15 eine Bibliothek in Form einer Datenbank abgelegt werden, welche Informationen über die kritischen Werte für unterschiedliche Personengruppen wie Kinder, Erwachsene, Männer und Frauen, sowie für unterschiedliche Körperkonstitutionen beinhaltet. Die Mikrocontroller-Einheit 15 kann außerdem eine Überwachung einer Einwirkdauer der Zugkräfte ermöglichen, die ebenfalls für den operativen Eingriff von hoher Relevanz ist.

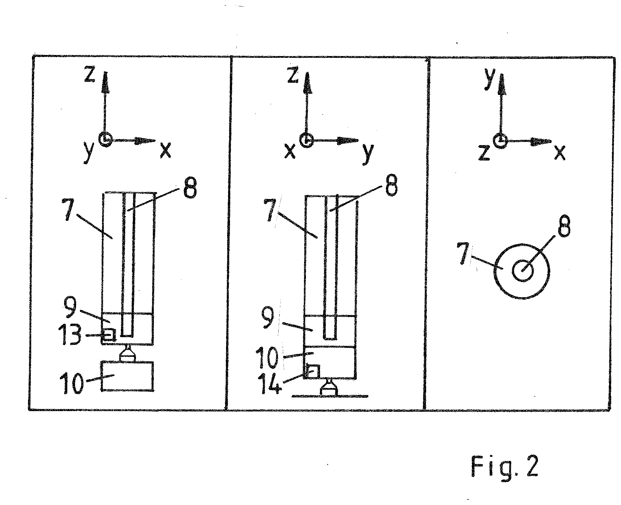

Figur 2 zeigt beispielhaft eine Stützvorrichtung 11, wie sie in der erfindungsgemäßen Beinzugvorrichtung zum Einsatz kommen kann. Die Stützvorrichtung 11 ist hierbei als Stützrolle ausgestaltet und in Figur 2 aus drei verschiedenen Perspektiven gezeigt, die jeweils durch das eingezeichnete Koordinatensystem angezeigt werden.

Das linke Teilbild zeigt hierbei einen Schnitt durch die Stützvorrichtung 11 in der xz-Ebene in Vorderansicht. Das mittlere Teilbild zeigt einen Schnitt in der yz-Ebene in Seitenansicht und das rechte Teilbild zeigt einen Schnitt in der xy-Ebene in Draufsicht.

Die in Figur 2 gezeigte Stützrolle der Stützvorrichtung 11 weist einen Kernstab 8 auf, der von einer Schaumstoffpolsterung 7 umgeben ist. Die Schaumstoffpolsterung 7 bildet also einen Zylinder, dessen Zylinderachse mit der Längsachse des Stabes 8 zusammenfällt. Die Stützrolle ist auf der Liegefläche 2 über zwei Führungsschlitten 9 und 10 gelagert. Dabei ist der untere zweite Führungsschlitten 10 im gezeigten Beispiel in y-Richtung bewegbar und der obere erste Führungsschlitten 9 in x-Richtung. Zusammen ermöglichen die Führungsschlitten 9 und 10 eine Bewegbarkeit der Stützrolle der Stützvorrichtung 11 in der xy-Ebene.

Im ersten Führungsschlitten 9 ist ein erster Kraftsensor 13 angeordnet, mit dem eine auf die Stützrolle der Stützvorrichtung 11 wirkende Kraft in x-Richtung messbar ist. Darüber hinaus weist der zweite Führungsschlitten 10 einen Kraftsensor 14 auf, mit dem eine Kraft in y-Richtung messbar ist.

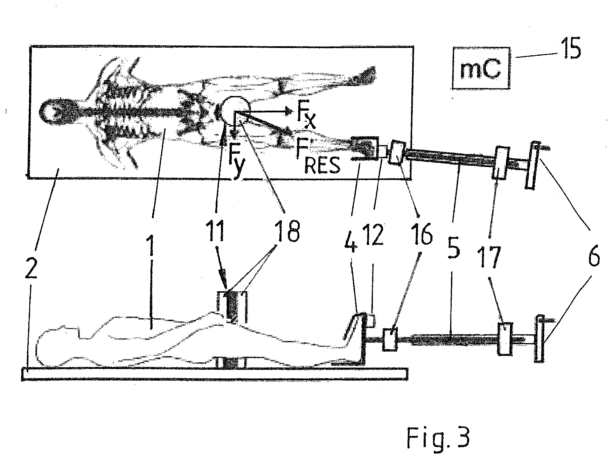

Figur 3 zeigt beispielhaft eine weitere mögliche Ausgestaltung einer erfindungsgemäßen Beinzugvorrichtung. Wiederum liegt ein Patient 1 auf einer Liegefläche 2, wobei zwischen seinen Oberschenkeln die Stützvorrichtung 11 angeordnet ist. Der rechte Fuß des Patienten 1 wird durch einen Extensionsschuh 4 gehalten, der wie in Figur 1 gezeigt einen Neigungssensor 12 aufweist. Auf den Extensionsschuh 4 kann mittels der Zugvorrichtung 5 durch Drehen einer Kurbel 6 eine Zugkraft auf das Bein des Patienten 1 ausgeübt werden. Insoweit entspricht die in Figur 3 gezeigte Vorrichtung der in Figur 1 gezeigten Vorrichtung.

Im in Figur 3 gezeigten Beispiel weist die Stützrolle der Stützvorrichtung 11 auf ihrer Zylindermantelfläche eine Druckmessfolie 18 auf, mit der ein durch den Patienten 1 auf die Stützrolle ausgeübter Druck ortsabhängig messbar ist. Die Druckmessfolie 18 kann zusätzlich oder alternativ zu der in Figur 2 gezeigten Kraftmessung vorgesehen sein.
Im in Figur 3 gezeigten Beispiel weist außerdem die Zugvorrichtung 5 einen Zugkraftsensor 16 auf, mit dem direkt die durch die Zugvorrichtung 5 ausgeübte Zugkraft auf dem Extensionsschuh 4 messbar ist. Der Zugkraftsensor 16 kann vorteilhaft alternativ zu der in Figur 2 gezeigten Kraftmessung an der Stützrolle vorgesehen sein. Der Zugkraftsensor 16 kann außerdem auch zusätzlich oder alternativ zur Druckmessfolie 18 eingesetzt werden.

Darüber hinaus weist die in Figur 3 gezeigte Zugvorrichtung 5 einen Momentsensor 17 auf, mit dem an der Zugspindel der Zugvorrichtung 5 ein Drehmoment erfasst werden kann, das in die anliegende Zugkraft umgerechnet werden kann. Auch hierdurch kann auf die am Patienten 1 wirkende Stützkraft geschlossen werden.

Es sei darauf hingewiesen, dass die in Figur 2 gezeigte Form der Kraftmessung, die Druckmessfolie 18, der Zugkraftsensor 16 und der Momentsensor 17 vorteilhafte Alternativen darstellen. Diese Sensoren 16;17;18 können zwar miteinander kombiniert werden, grundsätzlich ist jedoch eine dieser Messvorrichtungen erfindungsgemäß ausreichend.

## Patentansprüche

1. Beinzugvorrichtung aufweisend eine Zugvorrichtung (5) zum Ausüben einer Zugkraft auf ein Bein eines Patienten (1), eine Stützvorrichtung (11) und zwei zueinander senkrechte Führungsschlitten (9, 10) wobei die Stützvorrichtung (11) so gegenüber der Zugvorrichtung (5) anordbar ist, dass die Stützvorrichtung (11) den Patienten (1) gegen die von der Zugvorrichtung (5) ausgeübte Zugkraft abstützt, wobei die Stützvorrichtung (11) und/oder die Zugvorrichtung (5) zumindest einen Sensor aufweist, mit dem zumindest eine Kraft oder zumindest ein Druck messbar ist, die oder der von der Stützvorrichtung (11) durch Betätigung der Zugvorrichtung (5) auf den Patienten (1) ausgeübt wird und die Stützvorrichtung (11) mittels der zwei zueinander senkrechten Führungsschlitten (9;10) auf einer Liegefläche (2) lagerbar ist, wobei die Führungsschlitten (9;10) in jene Richtungen orientiert sind, in welche mit Kraftsensoren (13;14) die Kraftkomponenten messbar sind.

2. Beinzugvorrichtung nach dem vorhergehenden Anspruch, wobei die Stützvorrichtung (11) den zumindest einen Sensor (13;14;16;17;18) aufweist.

3. Beinzugvorrichtung nach einem der vorhergehenden Ansprüche, wobei der zumindest eine Sensor eine Druckmessfolie (18) ausweist oder ist, die auf einer bei Betätigung der Zugvorrichtung (5) auf den Patienten (1) drückenden Oberfläche der Stützvorrichtung (11) angeordnet ist.

4. Beinzugvorrichtung nach einem der vorhergehenden Ansprüche, aufweisend einen Liegefläche (2), wobei die Stützvorrichtung (11) zumindest zwei der Sensoren (13;14) aufweist, mit denen zwei zueinander senkrechte Kraftkomponenten in Richtung parallel zur Liegefläche (2) messbar sind.

5. Beinzugvorrichtung nach einem der vorhergehenden Ansprüche, aufweisend einen Liegefläche (2), wobei die Stützvorrichtung (11) eine Stützrolle aufweist, die senkrecht auf der Liegefläche (2) steht und die so angeordnet ist, dass sie zwischen den Oberschenkelknochen des Patienten (1) liegt, wenn mit der Zugvorrichtung (5) die Zugkraft auf das Bein des Patienten (1) ausgeübt wird und die Stützvorrichtung (11) den Patienten (1) gegen diese Zugkraft abstützt.

6. Beinzugvorrichtung nach einem des vorhergehenden Ansprüche, wobei die Zugvorrichtung (5) eine Fixierungsvorrichtung zur Fixierung eines Fußes des Patienten (1) aufweist, wobei an der Fixierungsvorrichtung ein Neigungssensor (12) angeordnet ist, mit dem die Lage der Fixierungsvorrichtung um zumindest eine Achse, vorzugsweise um drei Achsen, messbar ist.

7. Beinzugvorrichtung nach einem der vorhergehenden Ansprüche, wobei die Zugvorrichtung (5) eine Zugspindel aufweist, mit der die Zugkraft auf das Bein des Patienten (1) einstellbar ist.

8. Beinzugvorrichtung nach einem der vorhergehenden Ansprüche, aufweisend eine Mikrocontroller-Einheit (15), mit welcher durch die Kraft- oder Drucksensoren (13;14;16;17;18) und vorzugsweise auch den Neigungssensor (12) erzeugte Messungen auswertbar sind, und mit welcher bei Überschreiten kritischer Schwellenwerte für die mit den Sensoren (13;14;16;17;18) gemessenen Kräfte und/ oder für die mit den Sensoren (13;14;16;17;18) gemessenen Drücke und/oder für die mit dem Neigungssensor (12) gemessenen Neigungen ein Alarmsignal erzeugbar ist.

## Claims

1. Leg pulling device having a traction device (5) for exerting a pulling force on a leg of a patient (1), a supporting device (11), and two guide carriages (9, 10) perpendicular to each other, wherein the supporting device (11) can be arranged relative to the traction device (5) such that the supporting device (11) supports the patient (1) counter to the pulling force exerted by the traction device (5), wherein the supporting device (11) and/or the traction device (5) have/has at least one sensor, with which at least one force or at least one pressure exerted on the patient (1) by the supporting device (11) upon actuation of the traction device (5) can be measured, and the supporting device (11) can be mounted on a lying surface (2) by means of two mutually perpendicular guide carriages (9; 10), wherein the guide carriages (9; 10) are oriented in those directions in which the force components can be measured by force sensors (13; 14).

2. Leg pulling device according to the preceding claim, wherein the supporting device (11) has the at least one sensor (13; 14; 16; 17; 18).

3. Leg pulling device according to one of the preceding claims, wherein the at least one sensor has or is a pressure measuring film (18), which is arranged on a surface of the supporting device (11) pressing on the patient (1) upon actuation of the traction device (5).

4. Leg pulling device according to one of the preceding claims, having a lying surface (2), wherein the supporting device (11) has at least two of the sensors (13; 14), with which two mutually perpendicular force components in a direction parallel to the lying surface (2) can be measured.

5. Leg pulling device according to one of the preceding claims, having a lying surface (2), wherein the supporting device (11) has a supporting roller, which is upright on the lying surface (2) and which is arranged such that it is located between the femurs of the patient (1) when the pulling force is exerted on the leg of the patient (1) by the traction device (5), and the supporting device (11) supports the patient (1) counter to said pulling force.

6. Leg pulling device according to one of the preceding claims, wherein the traction device (5) has a fixing device for fixing a foot of the patient (1), wherein an inclination sensor (12), with which the position of the fixing device about at least one axis, preferably about three axes, can be measured, is arranged on the fixing device.

7. Leg pulling device according to one of the preceding claims, wherein the traction device (5) has a traction spindle, with which the pulling force on the leg of the patient (1) is adjustable.

8. Leg pulling device according to one of the preceding claims, having a microcontroller unit (15), with which measurements generated by the force or pressure sensors (13; 14; 16; 17; 18) and preferably also the inclination sensor (12) can be evaluated, and with which, when critical threshold values for the forces measured by the sensors (13; 14; 16; 17; 18) and/or for the pressures measured by the sensors (13; 14; 16; 17; 18) and/or for the inclinations measured by the inclination sensor (12) are exceeded, an alarm signal can be generated.

## Revendications

1. Dispositif de traction de jambe, présentant un dispositif de traction (5) permettant d'exercer une force de traction sur une jambe d'un patient (1), un dispositif de soutien (11) et deux chariots de guidage (9, 10) mutuellement perpendiculaires, dans lequel le dispositif de soutien (11) peut être disposé par rapport au dispositif de traction (5) de telle sorte que le dispositif de soutien (11) soutient le patient (1) contre la force de traction exercée par le dispositif de traction (5), dans lequel le dispositif de soutien (11) et/ou le dispositif de traction (5) présentent au moins un capteur qui permet de mesurer au moins une force ou au moins une pression qui est exercée sur le patient (1) par le dispositif de soutien (11) par l'actionnement du dispositif de traction (5), et le dispositif de soutien (11) peut être placé sur une surface de couchage (2) au moyen des deux chariots de guidage (9 ; 10) mutuellement perpendiculaires, les chariots de guidage (9 ; 10) étant orientés dans les directions dans lesquelles les composantes de force peuvent être mesurées par des capteurs de force (13 ; 14).

2. Dispositif de traction de jambe selon la revendication précédente, dans lequel le dispositif de soutien (11) présente au moins un capteur (13 ; 14 ; 16 ; 17 ; 18).

3. Dispositif de traction de jambe selon l'une quelconque des revendications précédentes, dans lequel ledit au moins un capteur présente ou est une feuille de mesure de pression (18) qui est disposée sur une surface du dispositif de soutien (11) plaquée contre le patient (1) lorsque le dispositif de traction (5) est actionné.

4. Dispositif de traction de jambe selon l'une quelconque des revendications précédentes, présentant une surface de couchage (2), le dispositif de soutien (11) présentant au moins deux des capteurs (13 ; 14) qui permettent de mesurer deux composantes de force mutuellement perpendiculaires dans une direction parallèle à la surface de couchage (2).

5. Dispositif de traction de jambe selon l'une quelconque des revendications précédentes, présentant une surface de couchage (2), le dispositif de soutien (11) présentant un rouleau de soutien qui est posé perpendiculairement sur la surface de couchage (2) et qui est disposé de telle sorte qu'il se trouve entre les fémurs du patient (1) lorsque le dispositif de traction (5) exerce la force de traction sur la jambe du patient (1) et le dispositif de soutien (11) soutient le patient (1) contre cette force de traction.

6. Dispositif de traction de jambe selon l'une quelconque des revendications précédentes, dans lequel le dispositif de traction (5) présente un dispositif d'immobilisation pour immobiliser un pied du patient (1), un capteur d'inclinaison (12) étant disposé sur le dispositif d'immobilisation et permettant de mesurer la position du dispositif d'immobilisation autour d'au moins un axe, de préférence autour de trois axes.

7. Dispositif de traction de jambe selon l'une quelconque des revendications précédentes, dans lequel le dispositif de traction (5) présente une broche de traction qui permet de régler la force de traction sur la jambe du patient (1).

8. Dispositif de traction de jambe selon l'une quelconque des revendications précédentes, présentant une unité de microcontrôleur (15) qui permet d'évaluer des mesures produites par les capteurs de force ou de pression (13 ; 14 ; 16 ; 17 ; 18) et de préférence aussi par le capteur d'inclinaison (12), et qui permet de produire un signal d'alarme en cas de dépassement de valeurs seuil critiques pour les forces mesurées par les capteurs (13 ; 14 ; 16 ; 17 ; 18) et/ou pour les pressions mesurées par les capteurs (13 ; 14; 16; 17 ; 18) et/ou pour les inclinaisons mesurées par le capter d'inclinaison (12).
